# EUROPÄISCHE PATENTSCHRIFT

(11) **EP 0 842 674 B1**
(45) Veröffentlichungstag und Bekanntmachung des Hinweises auf die Patenterteilung: **16.01.2002**
(21) Anmeldenummer: 97119693.6
(22) Anmeldetag: 11.11.1997
(51) Int. Cl.: A61M 27/00, A61G 7/05, A61F 5/44

(54) **Drainagesystem**
Draining system
Système de drainage

(30) Priorität: 15.11.1996 DE 29619897 U; 31.05.1997 DE 19722918
(43) Veröffentlichungstag der Anmeldung: 20.05.1998
(73) Patentinhaber: PRIMED HALBERSTADT MEDIZINTECHNIK GMBH, 38820 Halberstadt (DE)
(72) Erfinder: Neubauer, Norbert, 38820 Halberstadt (DE)
(74) Vertreter: Pfeiffer, Rolf-Gerd, Dipl.-Phys.

(56) Entgegenhaltungen:
- EP-A- 0 058 829
- DE-A- 3 233 944
- DE-C- 4 431 092
- FR-A- 2 231 576
- US-A- 4 055 176
- US-A- 4 573 965

## Beschreibung

Die Erfindung betrifft ein Drainagesystem, welches für vielfältige Drainageaufgaben in der Medizin, wie Chirurgie, Urologie, Traumatologie, Gynäkologie, insbesondere jedoch bei einer passiven Wunddrainage Verwendung findet.

Ein weit verbreitetes Drainagesystem ist das sogenannte Robinson-System, das im wesentlichen aus einem Auffangbeutel und einem daran anschließbaren Drainageschlauch besteht. Im Bereich des Auffangbeutels ist, wie bei anderen bekannten Systemen auch, ein in Richtung Drainageschlauch wirkendes Rückschlagventil vorgesehen. Die Nachteile dieses Systems bestehen in folgendem:
- die Steuerung des Anfließens der Drainageflüssigkeit nach dem Legen des Drainageschlauchs bedarf komplizierter Manipulationen,
- die Kontrolle der Durchgängigkeit des Drainageschlauchs ist kompliziert bis ausgeschlossen,
- durch das Bestehen einer praktisch geschlossenen Naßstrecke zwischen dem Auffangbeutel und dem Drainageschlauch besteht die erhebliche Gefahr einer rückläufigen Keimwanderung,
- die Sichtbarkeit des Drainageflusses ist nicht zu jedem Zeitpunkt gegeben.
Weiterhin verfügt dieses und andere bekannten Systeme über zwei innerhalb des Auffangbeutels im oberen Teil vorgesehene Aufhängeösen, wodurch eine erhöhte Abknickgefahr des Drainageschlauch besteht.

Diese Nachteile versucht ein Drainagesystem der Fa. BRAUN (unter der geschützten Bezeichnung "Ureofix" gehandelt) für den speziellen Verwendungszweck der Urinableitung teilweise zu verbessern. Zum einen sind dort genannte zwei Aufhängeösen aus dem Auffangbeutelbereich in eine bügelartige Ausbildung mit wiederum zwei Aufhängeösen heraus verlagert, was die Abknickgefahr des Drainageschlauches verringert. Als wesentliche Baugruppe ist bei dieser Lösung jedoch eine Auftrennung der Naßstrecke vorgesehen, was durch eine zwischen den Auffangbeutel und den Drainageschlauchanschluß eingebrachte steife Tropfkammer, welche innerhalb ein frei endendes Tropfrohr, das von einem weiteren Rohrstück beabstandet konzentrisch umfaßt wird, erreicht werden soll. Der Nachteil dieser Lösung besteht darin, daß sich ohne ständige Kontrolle auch in der vorgesehenen Tropfkammer eine unerwünscht hohe Drainageflüssigkeitsansammlung ausbilden kann.

Nachteilig bei allen bekannten Systemen ist weiterhin die relativ komplizierte Aufhängemöglichkeit der Auffangbeutel am Bett des Patienten, die darüber hinaus auch nur eine geringe Höhenänderung zuläßt, um die Schwerkraftsäule der Drainageflüssigkeit zu korrigieren.

Der Erfindung liegt die Aufgabe zugrunde, ein Drainagesystem anzugeben, das eine sichere Unterbrechung der Naßstrecke, eine gezielt vornehmbare Beeinflussung des Drainageflusses und dessen jederzeit mögliche Kontrolle und eine flexible Einstellung der Schwerkrafthöhe gewährleistet, ohne die Nachteile des Standes der Technik aufzuweisen.

Die Aufgabe wird durch die kennzeichnenden Merkmale des ersten Patentanspruchs gelöst. Vorteilhafte weitere Ausgestaltungen sind durch die nachgeordneten Ansprüche erfaßt.
Durch die erfindungsgemäße Ausbildung einer Tropfkammer wird:
- die bestehende Naßstrecke in effektiver Weise unterbrochen und damit wirkungsvoll eine Keimwanderung unterbunden,
- ein sofortiges Anfließen von Drainageflüssigkeit nach der operativen Plazierung eines Drains und eine Forcierung des Drainageflusses gewährleistet und
- eine jederzeit mögliche Kontrolle der Draindurchlässigkeit gewährleistet.
Eine erfindungsgemäß vorgesehene neue Aufhängemöglichkeit ermöglicht eine unkomplizierte Befestigung des gesamten Drainagesystems und insbesondere eine flexible Einstellung der Höhe einer Drainageflüssigkeitsäule zwecks Erzeugung eines kontinuierlichen Drainageflusses und verhindert die Gefahr eines Drainageschlauchabknickens.

Die Erfindung soll nachstehend anhand schematischer Ausführungsbeispiele näher erläutert werden. Es zeigen:
- Fig. 1: eine erstes grundsätzliches Ausführungsbeispiel in teilweise geschnittener Darstellung,
- Fig. 2: eine weitere Ausbildungsmöglichkeit einer Tropfkammer gemäß der Erfindung,
- Fig. 3: ein im Rahmen der Erfindung besonders bevorzugt zum Einsatz gelangendes Halteelement zur Fixierung des gesamten Drainagesystems,
- Fig. 4: eine Ausführungsform gemäß Fig. 1 in einer seitlichen Ansicht,
- Fig. 4a: eine mit einem Auffangbehälter verbundene und speziell angeordnete Lasche und
- Fig. 5: eine abgerüstete Ausführungsform, die in der Handhabbarkeit mit Nachteilen verbunden ist, ansonsten aber die gleichen vorteilhaften Wirkungen der Erfindung aufweist.

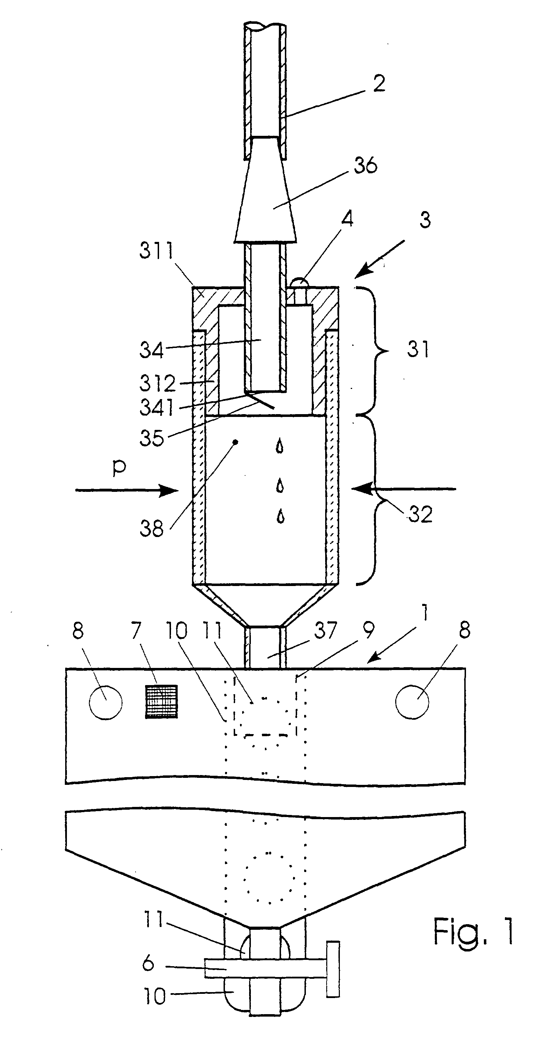

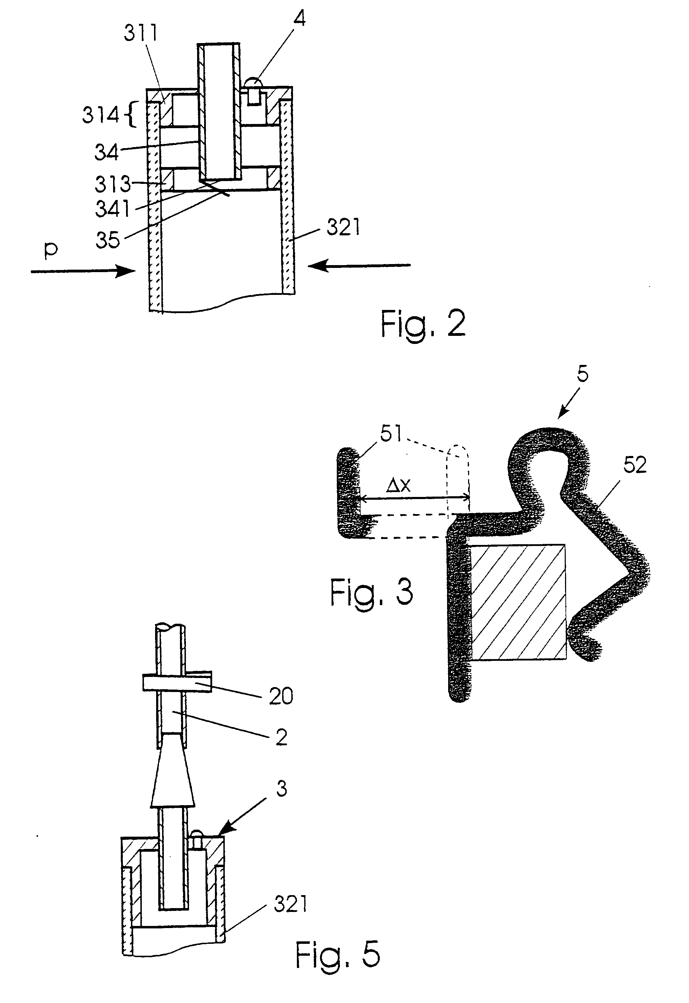

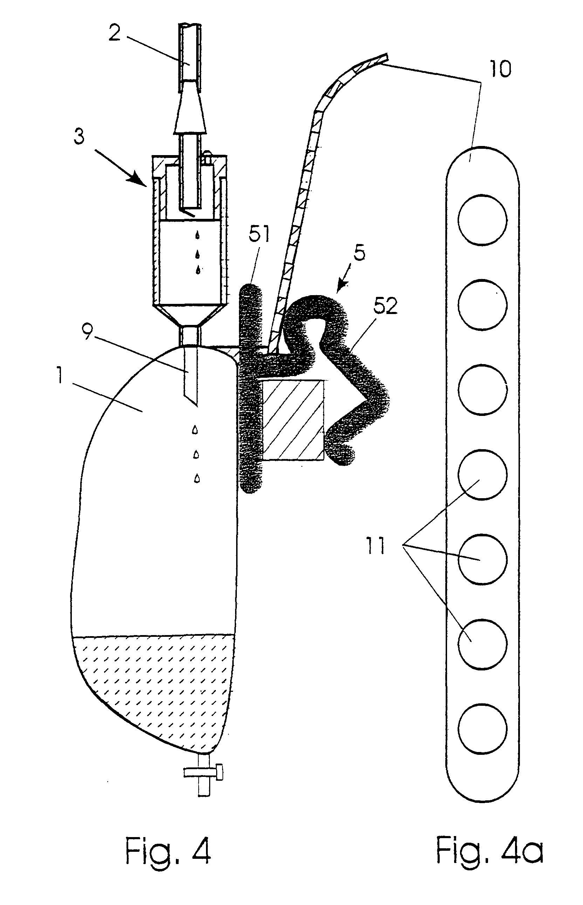

Ein im wesentlichen vollständiges Drainagesystem ist in Figur 1 dargestellt. Dabei zeigt Fig. 1 ein Endstück eines Drainageschlauches 2, der am nicht dargestellten Ende bereits operativ verlegt sein soll und am dargestellten Ende mit einer Tülle 36 verbunden ist, an die sich eine Tropfkammer 3 anschließt, von der aus Drainageflüssigkeit in einen Auffangbehälter in Form eines Beutels 1 gelangt. Die vorgesehene Tropfkammer 3 ist dabei so ausgebildet, daß sie einen biegesteifen Abschnitt 31 und einen in Richtung des Beutels 1 nachgeordneten flexiblen, mit einem äußeren Druck p beaufschlagbaren, formelastischen Abschnitt 32 aufweist. In einem Tropfkammerdeckel 311 ist ein Tropfrohr 34 koaxial derart eingebunden, daß sein in die Tropfkammer 3 hineinragendes Ende 34 in einer solchen Länge ausgeführt ist, daß eine Berührungsmöglichkeit mit der Innenwandung des flexiblen zweiten Abschnitts 32 auch dann ausgeschlossen ist, wenn der flexible Abschnitt mit einem äußeren Druck beaufschlagt wird, wobei sich Teile der Wandung des flexiblen Abschnitts 32 in Richtung zur Tropfkammerachse verschieben. Im in Fig. 1 dargestellten Beispiel ist das Tropfrohrende 341 hinter die Ebene des tropfkammerseitigen Endes einer Hülse 312 verlagert. Der Tropfkammerdeckel 311 und die Hülse 312 bilden bevorzugt eine einstückige Einheit. Ebenso liegt es im Rahmen der Erfindung, wie in Fig. 2 dargestellt, die Ausbildung der Abschnitte 31 und 32 so vorzunehmen, daß lediglich im Bereich des Tropfrohrendes 341 ein biegesteifer Ring 313 vorgesehen ist, wobei dieser Ring 313 und ein Stutzen 314 des Tropfkammerdeckels 311 von einem Schlauchstück 321 erfaßt sind. Bei dieser Ausführung sollte der Abstand zwischen dem Ring 313 und dem Tropfkammerdeckel 311 hinreichend gering gehalten werden. Andere Ausführung, die einen biegesteifen Abschnitt 31 und einen flexiblen Abschnitt 32 schaffen sind denkbar und liegen im Rahmen der Erfindung. Die Tropfkammer ist zumindest in Teilen durchsichtig ausgebildet, um den Drainagefluß überwachen zu können. Bevorzugt ist in jeder der dargestellten Ausführungsformen das tropfkammerseitige Ende 341 des Tropfrohres 34 mit einem Rückschlagventil 35 versehen. Weiterhin trägt der Tropfkammerdeckel einen hydrophoben Bakterienfilter für einen Druckausgleich, der weiter unten beschrieben wird. Als weiteres, im Rahmen der Erfindung wesentliches Merkmal, ist der Auffangbeutel 1 in einer Ebene zum auffangbehälterseitigen Tropfkammeranschluß 37 mit einem zentralsymmetrisch zu diesem angeordneten Aufhängemittel versehen, das bevorzugt als eine mit dem Beutel fest verbundene Lasche 10 ausgeführt ist, die wenigstens eine, insbesondere jedoch mehrere einander nachgeordnete Ösen 11 aufweist (vgl. Fig. 4a). Dem Gesamtsystem ist weiterhin ein Haltebügel 5 zugeordnet, der einerseits einen Haken 51 zur Aufnahme einer Öse 11 trägt und andrerseits einen federnden Klemmbügel 52 zur Anbringung des Gesamtsystems an einen Holm eines Patientenbettes aufweist. Der Haltebügel 5 weist bevorzugt weiterhin einen Bereich Δx auf, der in variabler Länge und insbesondere auch bis zu Δx = 0 (vgl. Fig. 4) ausführbar ist.
Im übrigen können für den Auffangbehälter 1 bekannte Auffangbeutel eingesetzt sein, die ein in sie integriertes Rückschlagventil 9, einen absperrbaren Ablaufhahn 6, weitere, im Rahmen der Erfindung ungenutzte Aufhängeösen 8, insbesondere jedoch eine Beutelentlüftung, die ebenfalls als hydrophobes Bakterienfilter 7 ausgeführt ist, aufweisen.

Das vorgeschlagene Drainagesystem soll im nachfolgenden in Funktion beschrieben werden. Ausgehend davon, daß der Drainageschlauch 2 bereits operativ gelegt wurde, wird auf den Bereich 32, als vorgesehener Quetschzone, manuell ein äußerer Druck ausgeübt. Dabei kann die in der Tropfkammer 3 vorhandene Luft durch das im Beutel 1 vorgesehene Rückschlagventil 9 und in geringer Weise auch über den Bakterienfilter 4 entweichen, während dessen das vorgesehene Rückschlagventil 35 verhindert, daß durch Luft bereits im Drainageschlauch 2 befindliches Sekret in die Wunde zurückgedrückt wird. Nach dem Lösen der äußeren Druckbeaufschlagung entsteht im flexiblen Bereich 32, durch sein Bestreben die Ausgangsform wieder einzunehmen, ein Unterdruck, das Rückschlagventil 35 öffnet sich und das Sekret wird angesaugt. Durch ein mehrmaliges Quetschen, vergleichbar mit einer Pumpe, kann der Unterdruck zu jedem Zeitpunkt erneut aufgebaut werden. Die vorgesehenen Bakterienfilter 4 und 7 sorgen im Gesamtsystem für den erforderlichen Druckausgleich, wodurch die Sekretflüssigkeit kontinuierlich in den Beutel 1 ablaufen kann, ohne daß sich in der Tropfkammer 3 ein steigender Flüssigkeitsspiegel ausbilden kann. Die oben beschriebene Ausbildung des biegesteifen Abschnitts 31 verhindert, daß in diesem Bereich eine deformierende Druckbeaufschlagung erfolgen kann, wodurch eine Kontaktierung des Tropfrohres 34 und des Rückschlagventils 35 mit Tropfkammerinnenwandungsbereichen verhindert wird. Damit ist eine effektive Luftsperre (Pasteur-Weg) für Bakterien gebildet, die Naßzone ist also durch echte Trockenkammerbereiche 38 unterbrochen und eine Keimstraße vermieden. Retrograde Keimwanderungen sind somit praktisch ausgeschlossen. Durch eine immer wiederholbare Druckbeaufschlagung des Bereichs 32 kann das Anlaufen der Drainageflüssigkeit in unkomplizierter Weise erfolgen und eine Verstopfungskontrolle des Drainageschlauchs 2 ist jederzeit auf einfache Weise möglich. Infolge der auf die im Drainageschlauch nach dem Ansaugen entstehende Flüssigkeitssäule wirkenden Schwerkraft, bildet sich ein kontinuierlicher Drainagefluß aus. Dabei ermöglicht die zentralsymmetrisch in einer Ebene zum auffangbehälterseitigen Tropfkammeranschluß 37 angeordnete Lasche 10 zum einen eine unkomplizierte Aufhängung des Gesamtsystems und vor allem eine variable Regulierung der Aufhängehöhe und damit eine Regulierung der Höhe der Schwerkraftsäule und damit des passiven Soges. Weiterhin vermeidet die zentrale Anbringung der Lasche 10 am Auffangbeutel 1 eine bestehende Abknickgefahr des Drainageschlauches.
Sollte in Ausnahmefällen auf das gemäß der Erfindung vorgesehene Rückschlagventil 35 verzichtet werden, die übrigen Vorteile der vorgeschlagenen Lösung, insbesondere das Funktionsprinzip, jedoch weiterhin genutzt werden, ist es unter Berücksichtigung von Handhabungsnachteilen auch möglich, eine Ausbildung, wie in Fig. 5 teilweise angedeutet, vorzusehen. Dabei ist der Drainageschlauch 2 mit einer Schlauchklemme 20 versehen, welche die Funktion des Rückschlagventils 35 übernimmt, wenn wie folgt verfahren wird: Die Schlauchklemme 20 wird geschlossen, anschließend ein Druck auf den Tropfkammerbereich 321 ausgeübt und erst danach die Schlauchklemme 20 geöffnet. Soll der Pumpvorgang mit seinen zu Fig. 1 beschriebenen Wirkungen hier wiederholt werden, muß danach die Schlauchklemme 20 erneut erst wieder verschlossen und wie beschrieben weiter vorgegangen werden.

### Bezugszeichenliste

- 1 -: Auffangbehälter (Beutel)
- 10 -: Aufhängemittel (Lasche)
- 11 -: Öse
- 2 -: Drainageschlauch
- 20 -: Schlauchklemme
- 3 -: Tropfkammer
- 31 -: biegesteifer erster Tropfkammerabschnitt
- 311 -: Tropfkammerdeckel
- 312 -: Hülse
- 313 -: biegesteifer Ring
- 314 -: Anschlußstutzen
- 32 -: flexibler, formelastischer zweiter Tropfkammerabschnitt
- 321 -: Schlauchstück zur Bildung des Abschnitts 32
- 34 -: Tropfrohr
- 341 -: Tropfrohrende
- 35 -: Rückschlagventil
- 36 -: Tülle
- 37 -: Tropfkammeranschluß (am Beutel 1)
- 38 -: Trockenkammerbereich
- 4, 7 -: Bakterienfilter (Entlüftung)
- 5 -: Haltebügel
- 51 -: Haken
- 52 -: federnder Klemmbügel
- 6 -: Ablaufhahn
- 8 -: Aufhängeösen
- 9 -: Rückschlagventil (im Beutel 1)
- p -: äußerer Druck

## Patentansprüche

1. Drainagesystem, bestehend aus einem Auffangbehälter (1), einem Drainageschlauch (2) und einer zwischen diesen angeordneten Tropfkammer (3), **dadurch gekennzeichnet, daß** die Tropfkammer (3) einen biegesteifen ersten Abschnitt (31) und einen diesem in Richtung des Auffangbehälters (1) nachgeordneten flexiblen, mit einem äußeren Druck (p) beaufschlagbaren, formelastischen zweiten Abschnitt (32) aufweist, der biegesteife Abschnitt (31) ein Tropfrohr (34) trägt, dessen in die Tropfkammer (3) hineinragendes Ende (341) in einer solchen Länge ausgeführt ist, daß eine Kontaktmöglichkeit mit dem flexiblen zweiten Abschnitt (32) unterbunden ist und am Auffangbehälter (1) ein zentralsymmetrisch in einer Ebene zum auffangbehälterseitigen Tropfkammeranschluß (37) angeordnetes Aufhängemittel (10) vorgesehen ist.

2. Drainagesystem nach Anspruch 1, **dadurch gekennzeichnet, daß** das Tropfrohr (34) an seinem Ende (341) mit einem in Richtung des Drainageschlauches (2) wirkenden Rückschlagventil (35) versehen ist.

3. Drainagesystem nach Anspruch 1, **dadurch gekennzeichnet, daß** der Drainageschlauch (2) am tropfkammereingangsseitigen Ende mit einer Schlauchklemme (20) zur Übernahme einer Rückschlagventilwirkung versehen ist.

4. Drainagesystem nach Anspruch 1, **dadurch gekennzeichnet, daß** der biegesteife Abschnitt (31) durch einen Tropfkammerdeckel (311) und eine Hülse (312) gebildet ist.

5. Drainagesystem nach Anspruch 4, **dadurch gekennzeichnet, daß** der Tropfkammerdeckel (311) und die Hülse (312) eine einstückige bauliche Einheit bilden.

6. Drainagesystem nach Anspruch 1, **dadurch gekennzeichnet, daß** der biegesteife Abschnitt (31) im wesentlichen durch einen Tropfkammerdeckel (311) und einen im Bereich des Tropfrohrendes (341) angeordneten biegesteifen Ring (313) gebildet sind, wobei sowohl der Ring (313) als auch ein am Tropfkammerdeckel (311) vorgesehener Anschlußstutzen (314) durch ein, den zweiten Abschnitt (32) bildendes Schlauchstück (321) erfaßt sind.

7. Drainagesystem nach einem der vorstehenden Ansprüche, **dadurch gekennzeichnet, daß** im Tropfkammerdeckel (311) ein hydrophober Bakterienfilter (4) zur Tropfkammerbelüftung vorgesehen ist.

8. Drainagesystem nach Anspruch 1, **dadurch gekennzeichnet, daß** das zentralsymmetrisch in einer Ebene zum auffangbehälterseitigen Tropfkammeranschluß (37) angeordnete Aufhängemittel durch eine mit dem Auffangbehälter (1) fest verbundene Lasche (10) gebildet ist, die in Nähe des Auffangbehälters (1) wenigstens eine Öse (11) aufweist.

9. Drainagesystem nach Anspruch 8, **dadurch gekennzeichnet, daß** der Lasche (10) mehrere, einander nachgeordnete Ösen (11) gegeben sind.

10. Drainagesystem nach Anspruch 1, 8 oder 9, **dadurch gekennzeichnet, daß** das die Ösen (11) aufnehmende und halternde Element ein Haken (51) eines Haltebügels (5) ist, der an dem, dem Haken (51) gegenüberliegenden Ende mit einem federnden Klemmbügel (52) versehen ist.

11. Drainagesystem nach Anspruch 10, **dadurch gekennzeichnet, daß** ein Abschnitt (Δx) des Haltebügels (5) in variabler Länge bis zu Δx = 0cm ausführbar ist.

## Claims

1. Drainage system comprising a catching vessel (1), a drain (2), and a drip chamber (3), said drip chamber being arranged between said vessel and said drain, **characterized in that** said drip chamber (3) is comprised of a first rigid portion (31) followed in direction of said catching vessel (1) by a second portion (32), said second portion (32) being flexible and elastic and is adapted to be subjected to an external pressure (p), said first rigid portion (31) supports a drip tube (34) being provided with an end portion (341) projecting into said drip chamber (3), the length of said end portion being so designed to eliminate any engagement with the second flexible portion (32), and **in that** a suspension means (10) is provided at the catching vessel (1), and a drip chamber connection (37) on the side of the catching vessel, said suspension means (10) being arranged in central symmetry to and in a common plane with the drip chamber connection (37).

2. Drainage system as claimed in claim 1, **characterized in that** the drip tube (34) is provided with a non-return valve (35) operative in direction of said drain (2) at its end portion (341).

3. Drainage system as claimed in claim 1, **characterized in that** the drain (2) is provided with a tubing clamp (20) at its end portion on the drip chamber inlet side, with the tubing clamp (20) taking over the function of a non-return valve.

4. Drainage system as claimed in claim 1, **characterized in that** the rigid portion (31) is comprised of a drip chamber lid (311) and a shell (312).

5. Drainage system as claimed in claim 4, **characterized in that** the drip chamber lid (311) and the shell (312) form a one-piece structural unit.

6. Drainage system as claimed in claim 1, **characterized in that** the rigid portion (31) is substantially comprised of a drip chamber lid (311) and a rigid ring (313), which is arranged in the range of the drip tube end portion (341), whereby the ring (313) as well as a connecting piece (314) provided at the drip chamber lid (311) are captured by a hose section (321), said hose section forming said second portion (32).

7. Drainage system as claimed in one of the preceding claims, **characterized in that** a hydrophobic bacterial filter is provided in the drip chamber lid (311) for ventilation of the drip chamber.

8. Drainage system as claimed in claim 1, **characterized in that** the suspension means (10), provided in central symmetry to and in a common plane with the drip chamber connection (37) located on the side of the catching vessel, is formed by a strap (10) attached to said catching vessel (1), said strap being provided with at least one eye (11) in the vicinity of the catching vessel (1).

9. Drainage system as claimed in claim 8, **characterized in that** the strap (10) is provided with a plurality of eyes (11) succeeding one another.

10. Drainage system as claimed in claims 1, 8 or 9, **characterized in that** the component for receiving and holding the eyes (11) is a hook (51) of a mounting clamp (5) which is, at its end portion in opposition to the hook (51), provided with a resilient clamping bow (52).

11. Drainage system as claimed in claim 10, **characterized in that** a section (Δx) of said mounting clamp (5) is of a variably designed length down to Δx = 0 cm.

## Revendications

1. Le système de drainage, comprenant une poche de recueil (1), un drain flexible (2) et un collecteur de gouttes (3) disposé entre ces deux composants, est **caractérisé en ce que** le collecteur de gouttes (3) comprend une première section rigide (31) et, disposée en aval dans le sens de la poche de recueil (1), une deuxième section élastique (32) susceptible d'être soumise à une pression extérieure (p) provoquant une déformation passagère avant de reprendre sa forme d'origine, que la section rigide (31) est doté d'un tube d'égouttage (34) dont la longueur de la portion (341) pénétrant dans l'espace intérieur du collecteur de gouttes (3) est telle que le tube ne puisse jamais entrer en contact avec la deuxième section flexible (32), que sur la poche de recueil (1) un dispositif d'accrochage en suspension (10) est disposé de manière symétrique centrale au niveau du raccord du collecteur de gouttes (37) côté poche de recueil.

2. Le système de drainage selon la revendication 1 est **caractérisé en ce que** le tube d'égouttage (34) est doté sur son extrémité (341) d'un clapet anti-retour (35) agissant en direction du drain flexible (2).

3. Le système de drainage selon la revendication 1 est **caractérisé en ce que**, sur le drain flexible (2), côté entrée dans le collecteur de gouttes, est prévue une crampe (20) pour tuyaux souples, destinée à accompagner l'effet du clapet anti-retour.

4. Le système de drainage selon la revendication 4 est **caractérisé en ce que** la section rigide (31) est formée par le couvercle du collecteur de gouttes (311) et de la douille (312).

5. Le système de drainage selon la revendication 2 est **caractérisé en ce que** le couvercle du collecteur de gouttes (311) et la douille (312) forme une entité d'une seul pièce.

6. Le système de drainage selon la revendication 1 est **caractérisé en ce que** la section rigide (31) est formée en principe par le couvercle du collecteur de gouttes (311) et un anneau rigide (313) disposé dans la zone à proximité de l'extrémité du tube d'égouttage (341), l'anneau (313) et une tubulure de raccord (314) prévue sur le couvercle du collecteur de gouttes (311) étant sous l'emprise d'un tuyau flexible (32) formant la deuxième section.

7. Le système de drainage selon une des revendications précédentes est **caractérisé en ce qu'**à l'intérieur du couvercle (311) du collecteur de gouttes, un filtre antibactérien hydrophobe (4) est installé pour assurer l'aération du collecteur de goutte.

8. Le système de drainage selon la revendication 1 est **caractérisé en ce que** le dispositif d'attache en suspension est disposé de manière symétrique centrale au niveau du raccord ( 37) au collecteur de gouttes, côté poche de recueil, et forme une languette (10) solidaire de la poche de recueil (1) et comprenant à proximité de la poche de recueil, (1) au moins un oeillet (11).

9. Le système de drainage selon la revendication 8 est **caractérisé en ce que** la languette (10) est dotée de plusieurs oeillets consécutifs (11).

10. Le système de drainage selon les revendications 1, 8 ou 9 est **caractérisé en ce que** l'élément recevant et maintenant les oeillets (11) en place prend la forme d'un crochet (51) et d'un étrier (5) dont l'extrémité opposée au crochet est pourvu d'un étrier de serrage élastique (52).

11. Le système de drainage selon la revendication 10 est **caractérisé en ce que** la section (Δx) de l'étrier (5) peut être exécutée dans des longueurs variables allant jusqu'à Δx =0cm.
